# EUROPEAN PATENT SPECIFICATION

(11) **EP 3 423 007 B1**
(45) Date of publication and mention of the grant of the patent: **07.12.2022**
(21) Application number: 17710032.8
(22) Date of filing: 27.02.2017
(51) Int. Cl.: A61F 2/30, A61F 2/38

(54) **PROSTHESIS**
PROTHESE
PROTHÈSE

(30) Priority: 02.03.2016 GB 201603606
(43) Date of publication of application: 09.01.2019
(73) Proprietor: Invibio Knees Limited, Thornton Cleveleys, Lancashire FY5 4QD (GB)
(72) Inventor: BRISCOE, Adam, Thornton Cleveleys Lancashire FY5 4QD (GB); JOHNS, Ian, Thornton-Cleveleys Lancashire FY5 4QD (GB)
(74) Representative: Thompson, Nicola Ruth
(86) International application number: PCT/GB2017/050520
(87) International publication number: WO 2017/149279

(56) References cited:
- EP-A1- 1 836 997
- US-A1- 2008 140 214
- US-A1- 2010 102 484
- US-A1- 2012 256 344

## Description

This invention relates to a prosthesis and particularly, although not exclusively, relates to a knee prosthesis. Preferred embodiments relate to a femoral component of a knee prosthesis, a combination and assembly which includes said femoral component and a method of making a femoral component. Document US 2010/102484 A1 defines the preamble of claim 1. Various materials have been proposed and/or used for the femoral and tibial components of knee prostheses. For example, the components may be made from various combinations of metal, ceramics and polymers. In the last ten years, more interest has been focussed on all-polymer knee prostheses. For example, US2009/0164023 (Devine) described artificial joints, for example knee joints, which include both bearing surfaces made from composite materials which comprise a polyaryletherketone polymer (e.g. polyetheretherketone i.e. PEEK) and carbon fibre. The composite materials are said to have improved wear compared to other combinations based on metal bearing on metal, metal bearing on polymer or one specified polymer bearing on another specified polymer.

US2010/0312348 (Wang) discloses an orthopaedic prosthetic joint which may be a knee joint. The joint is said to comprise a first bearing surface made of a polyaryletherketone (PAEK), for example PEEK, and a second joint component having a second bearing surface made of a polymer which is softer than the PEEK. In preferred embodiments, the second polymer is an ultra-high molecular weight polyethylene (UHMWPE).

Both US2009/0164023 and US2010/0312348 suggest the bearing surfaces may be provided in various ways. For example US2009/0164023 suggests components may be made substantially entirely from the composite materials described or only a bearing surface may be formed from the composite materials, for example by capping or coating a layer of composite material on a precursor, for example defining a femoral head, which may be made from metal or ceramic. No further detail on how the components may be made is provided.

Similarly, US2010/0312348 describes, for example, a PEEK bearing and states this may be a stand-alone PEEK component or a PEEK layer could be coated, moulded or grafted onto another solid or porous polymer or polymeric composite; or onto a solid or porous metallic or ceramic substrate. Again, other than generic statements, no further detail or specific embodiments on how the components may be made is provided.

Applicant has appreciated that techniques that may be used for manufacturing components of knee prostheses, may affect wear and longevity of the prostheses in use. It is an object of the present invention to address this problem.

Preferred embodiments aim to provide an advantageous femoral component of a knee prosthesis. Preferred embodiments aim to provide an advantageous knee prosthesis. According to a first aspect of the invention, there is provided a femoral component of a knee prosthesis as defined in claim 1, said component comprising a curved outer surface for bearing against a tibial component, said curved outer surface including a posterior end and an anterior end, wherein said curved outer surface includes an area (A) which extends from a first position closer to the posterior end to a second position closer to the anterior end.

Area (A) includes no remnant of a parting line. A parting line (or seam) suitably comprises a line formed on an injection moulded part, during injection moulding, which witnesses where two mould parts met. On ejection from a mould, a parting line may be in the form of a thin line extending from a surface of the injection moulded part. The line may typically have a height of 0.02-0.03mm.. As described, area (A) preferably does not include any parting line and preferably does not include any remnant of a parting line. Thus, area (A) preferably does not include any parting line and never included any parting line - i.e. a parting line has not been removed to define any part of area (A). Thus, area (A) suitably is an area of said curved outer surface of said femoral component which has not been treated to remove or reduce any parting line. This may be advantageous since no machining (or the like) needs to be used to define area (A), thereby avoiding production of machining marks or contamination of area (A) by metal (or other) particles detached from a machine tool, for example a metal machine tool. Furthermore, it may reduce manufacturing cost by avoiding a potentially extra precision machining step in producing the femoral component.

The shape of area (A) is preferably wholly defined by a tool surface used in its manufacture, for example by a surface of an injection moulding tool. Area (A) is preferably a wholly as-moulded surface. Other than being cleaned and/or sterilised, it is preferably not a surface which has been subjected to any treatment which may change its shape.

Preferably, said femoral component includes said curved outer surface for bearing against a tibial component and a flexion/extension axis, suitably determined as described in ISO14243-1:2009(E) at 3.6.

Area (A) of said curved outer surface suitably subtends an angle (e.g. a maximum angle) of at least 150°, preferably at least 160°, with the flexion/extension axis. That is, an angle defined between said first and second positions of said area and said flexion/extension axis is preferably as stated. The angle subtended is suitably defined in a plane which is suitably perpendicular to the flexion/extension axis. The plane may extend through a condyle of the femoral component. Said angle is suitably 180° or less.

Area (A) suitably comprises an area of the curved outer surface which is arranged to contact, pivot and/or roll over a surface (herein a "tibial surface") of a tibial component in normal use to define at least part of a knee prosthesis. The femoral component is arranged to pivot and/or roll over the tibial surface so the femoral component can pivot through an angle of at least 140°, for example at least 150° or at least 155°, with only area (A) of said femoral component (and suitably no other area of the femoral component) contacting the surface of the tibial component. Thus, said femoral component is arranged to pivot through an angle of at least 140°, for example at least 150° or at least 155°, without any parting line and/or without any remnant of a parting line contacting the tibial surface. It may be arranged to pivot through an angle of less than 180°, for example less than 175° or less than 170°.

As described, area (A) suitably comprises an area of the curved outer surface which is arranged to contact and roll over a tibial surface in use. Movement of area (A) on a tibial surface may be affected by the shape of the tibial surface. To address this, features of the femoral component may be assessed in conjunction with a planar surface, for example, as described in Assessment (A) hereinafter. Preferably, the femoral component is arranged such that area (A) can contact a planar surface and be pivoted through an angle of at least 140° (preferably through at least 150° or 155°) without any parting line, remnant of a parting line or area from which a parting line has been removed, contacting the planar surface. It may be arranged to pivot through an angle of less than 180°, for example less than 175° or less than 170°.

The femoral component may be subjected to the following assessment:
(i) contact a planar surface with area (A) of said curved outer surface of said femoral component, wherein area (A) suitably represents an area of the curved outer surface which is arranged to contact and roll over a tibial surface in normal use; (ii) pivoting (suitably about an axis parallel to the planar surface) the femoral component so that area (A) rolls over the planar surface between first and second extreme positions, wherein during said pivoting movement of the femoral component between said extreme positions, no parting line, remnant of a parting line and/or other area from which a parting line has been removed contacts the planar surface; and (iii) assessing the maximum angle through which the femoral component can be pivoted between said first and second extreme positions.

The femoral component can be pivoted through an angle of at least 140°, preferably at least 145°, more preferably at least 150°, especially at least 155°,without any contact of a parting line, remnant of a parting line and/or other area from which a parting line has been removed with the planar surface. Said angle through which said femoral component may be pivoted as aforesaid may be less than 180°, less than 175° or less than 170°.

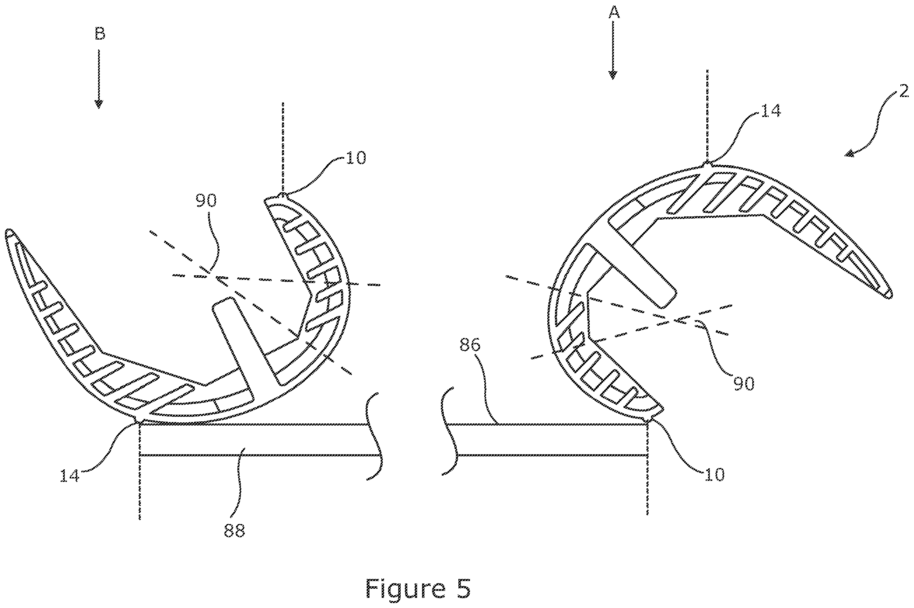

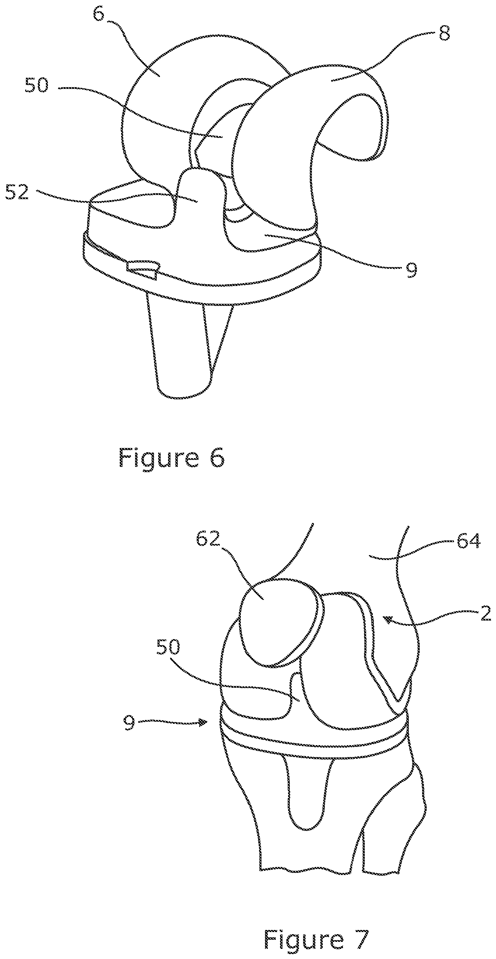

Said first extreme position of the femoral component may represent an extreme of flexion of the femoral component (e.g. wherein an area of the femoral component adjacent its posterior end contacts the planar surface, for example as shown in Figure 5, right hand drawing and Figure 6). Said first extreme position may involve the curved outer surface contacting the planar surface relatively close to the posterior end of the curved outer surface. For example, it is suitably possible only to pivot the femoral component, from said first extreme position, through a small angle x on the planar surface before the posterior end of the curved outer surface contacts the planar surface. The angle x suitably represents the angle through which the femoral component may be pivoted between the extreme of flexion and a position wherein the posterior end (which is outside area (A) and may include a parting line or remnant of a parting line) contacts the planar surface. For example, referring to Figure 5, right hand drawing, the femoral component can only be rotated clockwise through a small angle before posterior end 97 of the curved surface contacts the planar surface. Angle x is preferably less than 10°, for example less than 5°.

Said second extreme position may represent an extreme of extension of the femoral component, for example as shown in Figure 5, left hand drawing. Said second extreme position may involve the curved outer surface contacting the planar surface a substantial distance from the posterior end of the curved outer surface. In fact, it may involve the curved outer surface contacting the planar surface at a position on the curved outer surface which is closer to an anterior end (e.g. 99 in Figure 5) of the curved outer surface than to said posterior end. For example, it is suitably possible to pivot the femoral component, from the second extreme position, through an angle y on the planar surface before the anterior end of the curved outer surface contacts the planar surface. The angle y suitably represents the angle through which the femoral component may be pivoted on the planar surface between the second extreme position and a position wherein the anterior end (which is outside area (A)) contacts the planar surface. For example, referring to Figure 5, left hand drawing, the femoral component can be rotated anti-clockwise through a relatively large angle before anterior end 99 of the curved surface contacts the planar surface. Angle y may be greater than 20°, 30°, 40° or 50°. The ratio of y divided by x may be at least 3, at least 5, at least 10 or at least 20. It may be less than 70.

During the movement of the femoral component from said first extreme position to said position wherein the posterior end contacts the planar surface (e.g. clockwise from the position shown in Figure 5, right hand drawing), the femoral component may travel a linear distance LD1 across the planar surface LD1 may be less than 10mm, preferably less than 5mm LD1 may be at least 0.5mm, for example at least 1mm.

During the movement of the femoral component from the second extreme position to said position wherein the anterior end contacts the planar surface (e.g. anti-clockwise from the position shown in Figure 5, left hand drawing), the femoral component may travel a linear distance LD2 across the planar surface. LD2 may be at least 20mm, preferably at least 40mm.

The ratio of LD2 divided by LD1 may be at least 5, for example at least 7, 9 or 12.

Said femoral component may include a parting line, a remnant of a parting line or an area from which a parting line has been removed. When it includes a parting line, said parting line may have a height of less than 0.05mm, for examples less than 0.03mm. The height may be at least 0.005mm, for example at least 0.01mm. The height is typically 0.025mm.

Said femoral component may include a parting line, a remnant of a parting line or an area from which a parting line has been removed in a region of said curved outer surface of the femoral component which is outside area (A) and is arranged between area (A) and a posterior end of the femoral component.

Said femoral component may include a parting line, a remnant of a parting line or an area from which a parting line has been removed in a region of said curved outer surface of the femoral component which is outside area (A) and is arranged between area (A) and an anterior end of the femoral component.

Said femoral component preferably includes first and second condyles which are suitably arranged to engage and/or roll over the tibial surface and/or the planar surface described during assessment of the femoral component.

Said femoral component preferably includes an undercut region. Said undercut region is preferably defined in a surface of said femoral component which faces in a direction which is opposite to the direction in which said outer surface faces. Said undercut region is preferably an internal undercut. Said undercut region is preferably arranged to define a cement pocket in said femoral component for retaining cement which may be used to facilitate securement of the femoral component to a femur during implantation. Said cement pocket may have a depth of at least 0.5mm, more preferably at least 1mm. The cement pockets may be less than 8mm.

Said femoral component preferably includes multiple undercut regions each of which may have any feature of said undercut region described. Said femoral component suitably includes a series of ribs (which may define one or more undercut regions) defined in said surface of the femoral component which faces in a direction which is opposite to the direction in which said outer surface faces. Preferably, the ribs are arranged mutually parallel to each other, having straight sides. The ribs are equi-distantly spaced. Preferably, the ribs run parallel to the flexion extension axis.

Said femoral component preferably includes undercut regions associated with anterior and/or posterior flanges thereof.

Said femoral component comprises an injection moulded component made by injection moulding. Said femoral component preferably comprises a polymeric material, for example a thermoplastic polymeric material. At least 50wt%, suitably at least 70wt%, preferably at least 80wt%, more preferably 90wt%, especially at least 95wt%, for example at least 99wt%. of said femoral component is made from thermoplastic polymeric material, for example from a first polymer as herein described.

Said curved outer surface of said femoral component is preferably formed in an injection moulding process. Said curved outer surface preferably comprises a polymeric material, for example a thermoplastic polymeric material. At least 50wt%, suitably at least 70wt%, preferably at least 80wt%, more preferably 90wt%, especially at least 95wt%, for example at least 99wt%. of said curved outer surface is made from thermoplastic polymeric material, for example from a first polymer described herein.

Said femoral component is preferably a solid body. It is preferably monolithic. It is preferably made in one piece by injection moulding.

Said first polymer is preferably a polyaryletherketone. A preferred polyaryletherketone has a repeat unit of formula (I) 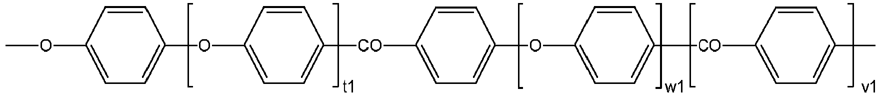 where t1 and w1 independently represent 0 or 1 and v1 represents 0, 1 or 2.

Said polyaryletherketone suitably includes at least 90, 95 or 99 mol % of repeat unit of formula I. Said polyaryletherketone suitably includes at least 90, 95 or 99wt% of repeat units of formula I.

Said polyaryletherketone preferably consists essentially of a repeat unit of formula I. Preferred polymeric materials comprise (especially consist essentially of) a said repeat unit wherein t1=1, v1=0 and w1=0; t1=0, v1=0 and w1=0; t1=0, w1=1, v1=2; or t1=0, v1=1 and w1=0. More preferred comprise (especially consist essentially of) a said repeat unit wherein t1=1, v1=0 and w1=0; or t1=0, v1=0 and w1=0. The most preferred comprises (especially consists essentially of) a said repeat unit wherein t1=1, v1=0 and w1=0.

In preferred embodiments, said first polymer is selected from polyetheretherketone, polyetherketone, polyetherketoneetherketoneketone and polyetherketoneketone. In a more preferred embodiment, said first polymer is selected from polyetherketone and polyetheretherketone. In an especially preferred embodiment, said first polymer material is polyetheretherketone.

Said polyaryletherketone may have a Notched Izod Impact Strength (specimen 80mm x 10mm x 4mm with a cut 0.25mm notch (Type A), tested at 23°C, in accordance with ISO180) of at least 4KJm⁻², preferably at least 5KJm⁻², more preferably at least 6KJm⁻². Said Notched Izod Impact Strength, measured as aforesaid, may be less than 10KJm⁻², suitably less than 8KJm⁻².. The Notched Izod Impact Strength, measured as aforesaid, may be at least 3KJm⁻², suitably at least 4KJm⁻², preferably at least 5KJm⁻². Said impact strength may be less than 50 KJm⁻², suitably less than 30KJm⁻².

Said polyaryletherketone suitably has a melt viscosity (MV) of at least 0.06 kNsm⁻², preferably has a MV of at least 0.09 kNsm⁻², more preferably at least 0.12 kNsm⁻², especially at least 0.15 kNsm⁻². Advantageously, the MV may be at least 0.35 kNsm⁻² and especially at least 0.40 kNsm⁻² An MV of 0.45 kNsm⁻² has been found to be particularly advantageous in the manufacture of accurate, strong frameworks.

Unless otherwise stated herein, MV is measured using a Bohlin Instruments RH2000 capillary rheometer according to ISO 11443 operating at 340°C and a shear rate of 1000s⁻¹ using a 0.5mm (capillary diameter) x 8.0mm (capillary length) die with entry angle 180°C. Granules are loaded into the barrel and left to pre-heat for 10 minutes. The viscosity is measured once steady state conditions are reached and maintained, nominally 5 minutes after the start of the test. Said polyaryletherketone may have a MV of less than 1.00 kNsm⁻², preferably less than 0.5 kNsm⁻². Said polyaryletherketone may have a MV in the range 0.09 to 0.5 kNsm⁻², preferably in the range 0.14 to 0.5 kNsm⁻², more preferably in the range 0.4 to 0.5 kNsm⁻².

Said polyaryletherketone may have a tensile strength, measured in accordance with ISO527 (specimen type 1b) tested at 23°C at a rate of 50mm/minute of at least 20 MPa, preferably at least 60 MPa, more preferably at least 80 MPa. The tensile strength is preferably in the range 80-110 MPa, more preferably in the range 80-100 MPa.

Said polyaryletherketone may have a flexural strength, measured in accordance with ISO178 (80mm x 10mm x 4mm specimen, tested in three-point-bend at 23°C at a rate of 2mm/minute) of at least 50 MPa, preferably at least 100 MPa, more preferably at least 145 MPa. The flexural strength is preferably in the range 145-180MPa, more preferably in the range 145-164 MPa.

Said polyaryletherketone may have a flexural modulus, measured in accordance with ISO178 (80mm x 10mm x 4mm specimen, tested in three-point-bend at 23°C at a rate of 2mm/minute) of at least 1 GPa, suitably at least 2 GPa, preferably at least 3 GPa, more preferably at least 3.5 GPa. The flexural modulus is preferably in the range 3.5-4.5 GPa, more preferably in the range 3.5-4.1 GPa.

Said polyaryletherketone may be amorphous or semi-crystalline. It is preferably crystallisable. It is preferably semi-crystalline. The level and extent of crystallinity in a polymer is preferably measured by wide angle X-ray diffraction (also referred to as Wide Angle X-ray Scattering or WAXS), for example as described by Blundell and Osborn (Polymer 24, 953, 1983). Alternatively, crystallinity may be assessed by Differential Scanning Calorimetry (DSC). The level of crystallinity of said polyaryletherketone may be at least 1%, suitably at least 3%, preferably at least 5% and more preferably at least 10%. In especially preferred embodiments, the crystallinity may be greater than 25%. It may be less than 50% or less than 40%. The main peak of the melting endotherm (Tm) of said polyaryletherketone (if crystalline) may be at least 300°C.

Said femoral component is preferably sterile. Same femoral component is preferably provided in a sterile package.

According to a second aspect of the invention, there is provided a combination for a knee prosthesis, the combination comprising a femoral component according to claim 1 and a tibial component. Any rolling movement of the femoral component suitably represents the normal and/or intended movement of the femoral component relative to the tibial component - that is, the normal and/or intended movement when the combination is implanted in a human body. Preferably, during such movement, no parting line (e.g. which is part of said curved surface of the femoral component), remnant of a parting line, (e.g. which is part of said curved surface of the femoral component) or area (e.g. which is part of said curved surface of the femoral component) from which a parting line has been removed, contacts the tibial surface.

Said angle may be less than 180°, less than 175° or less than 170°.

The femoral component may be arranged to roll through an angle up to at least 150° or up to at least 160° without any parting line, remnant of a parting line or area from which a parting line has been removed contacting the tibial surface. Said femoral component may be arranged to roll on the tibial surface between a first extreme of movement (e.g. wherein, when implanted, the knee prosthesis is at one extreme of normal flexion) and a second extreme of movement (e.g. wherein, when implanted, the knee prosthesis is at one extreme of normal extension) without any contact with the tibial surface of any parting line, remnant of a parting line or area from which a parting line has been removed. By avoiding any such contact with the tibial surface during normal flexion and normal extension of the knee prosthesis, wear on the tibial surface (which in a preferred embodiments is softer than said curved outer surface) may be minimised.

Said femoral component of the second aspect may have any feature of the femoral component of the first aspect.

Said tibial component preferably comprises an injection moulded component. Said tibial component is preferably made substantially entirely by injection moulding. Said tibial component preferably comprises a polymeric material, for example a thermoplastic polymeric material. At least 50wt%, suitably at least 70wt%. preferably at least 80wt%, more preferably at least 90wt%, especially at least 95wt% of said tibial component is made from thermoplastic polymeric material, for example from a second polymer described herein.

Said tibial surface of said tibial component is preferably formed in an injection moulding process. Said tibial surface preferably comprises a polymeric material, for example a thermoplastic polymeric material. At least 50wt%, suitably at least 70wt%. preferably at least 80wt%, more preferably at least 90wt%, especially at least 95wt% of said tibial surface is made from thermoplastic polymeric material, for example from a second polymer described herein.

Said tibial component is preferably a solid body. It is preferably monolithic. It is preferably made in one-piece by injection moulding.

Said femoral component preferably comprises a first polymer as described and said tibial component comprises a second polymer as described. Preferably, said first polymer is harder than said second polymer.

Unless otherwise stated herein, the relative hardness of the materias of said first and second polymers may be assessed by the Ball indentation method described in ISO 2039-1 : 2001.

A hardness ratio may be defined as the hardness of the first polymer divided by the hardness of the second polymer. The hardness ratio may be at least 2, 3, 4, 5 or 6. It may be less than 10, 9 or 8. It is suitably in the range 4 to 9.

Said first polymer may be a polyaryletherketone (PAEK), as described. Said second polymer may be a polyolefin, for example polyethylene, a polyurethane or a polyamide. Said second polymer is preferably polyethylene. Said polyethylene may be crosslinked. It is preferably crosslinked, for example by irradiation. It may comprise UHMWPE. Preferably, it comprises UHMWPE which has been crosslinked at least three times by irradiation. It may comprise X3^{™} UHMWPE of Stryker Corporation, crosslinked as described in US 7517919.

According to a third aspect of the invention, there is provided an assembly comprising a femoral component bearing against a tibial component, said femoral component and/or tibial component being as described in the first and/or second aspects.

According to a fourth aspect of the invention, there is provided a method of making a femoral component according to the first aspect and/or second aspect which comprises injection moulding a thermoplastic polymeric material, for example comprising said first polymer, as described according to said first aspect, thereby to form said femoral component.

According to a further aspect of the invention, there is provided a tooling apparatus configured for moulding a femoral component, the tooling apparatus comprising a mould for injection moulding the femoral component, the mould having a first element, a second element, a third element, and at least one up and away element, wherein the mould is operable such that parting lines are formed at locations on the surface of the component which do not obstruct use, or cause damage to a corresponding mating surface.

Said femoral component suitably includes a curved outer surface for bearing against the tibial component, wherein said curved outer surface includes a posterior end and an anterior end, wherein said curved outer surface includes an area (A) which extends from a first position close to the posterior end to a second position closer to the anterior end, wherein during injection moulding of said thermoplastic polymeric material no parting line is produced within area (A). During said injection moulding, a parting line may be produced on said curved outer surface outside area (A). For example, a parting line may be produced, on said curved outer surface, between area (A) and a posterior end of the femoral component; and/or a parting line may be produced, on said curved outer surface, between area (A) and an anterior end of the femoral component.

The invention may be used in a method of providing a knee prosthesis, the method comprising implanting a femoral component according to the first aspect and/or as described in the second and/or third aspects into a human body. The method may comprise implanting a tibial component as described.

Specific embodiments of the invention will now be described, by way of example, with reference to the accompanying drawings, in which:
Figure 1 is a schematic side elevation of a femoral component;
Figure 2 is a schematic vertical cross-section through a femoral component of Figure 1.
Figure 3 is a schematic perspective view of the femoral component in the general direction of arrow III of Figure 1 to show an inside surface of the component;
Figure 4 is a schematic perspective view of the femoral components of Figure 1 focussing on an outside surface of the component;
Figure 5 is a schematic showing a femoral component (in vertical cross-section taken through its axis of rotation) contacting a planar surface and illustrating extreme positions of the femoral component as it is on the planar surface.
Figure 6 is a schematic representation of a typical prosthetic knee assembly including a femoral component in its fully flexed condition;
Figure 7 is a schematic representation of a prosthetic knee secured to a femur and tibia and showing a patella;
Figure 8 is a schematic front view from an anterior end of part of the curved outer surface of the femoral component;
Figure 9 is a schematic vertical cross-section through an injection moulding tool;
Figure 10 is a schematic cross-section through the tool during disassembly of part of the shell of the tool;
Figure 11 is a schematic cross-section through the tool after removal of part of the outer shell; and
Figure 12 is a view similar to Figure 3 except that a femoral component of a posterior stabilised knee is shown.

In the Figures, the same or similar parts are annotated with the same reference numerals.

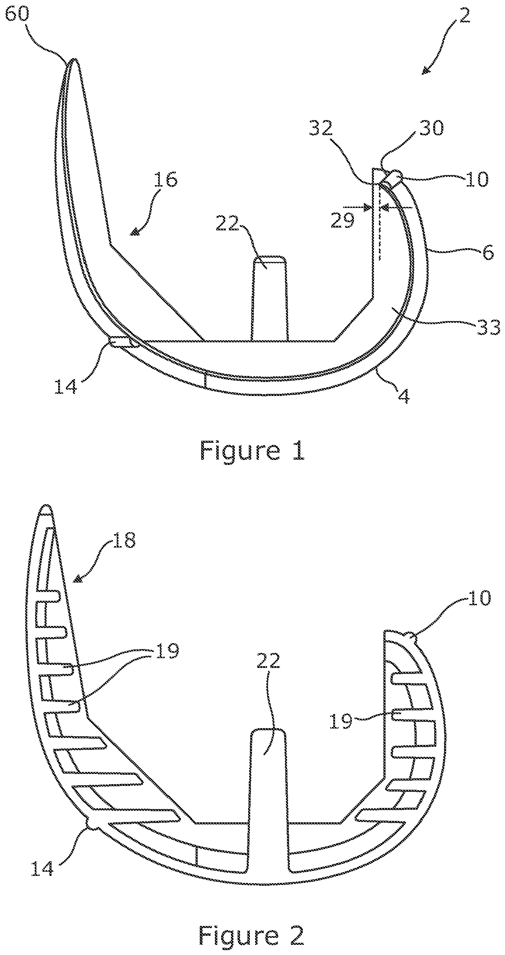

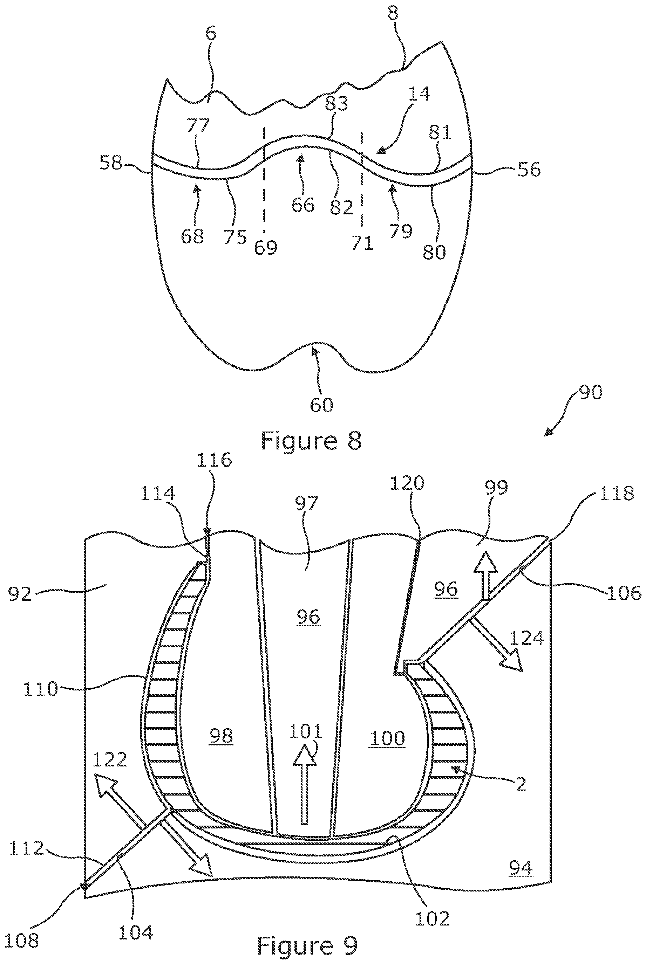

A femoral component 2 (shown in Figure 1 to 4) of a prosthetic knee assembly comprises a one-piece, monolithic injection moulding. The component 2 includes a curved outer surface 4 which comprises a first condyle 6 and a second condyle 8. The first and second condyles 6, 8 define an articulation surface of the femoral component and are arranged to contact an articulation surface of a tibial component 9 shown in Figures 6 and 7. A bearing surface representing an articulation surface of a tibial component is represented in Figure 5 (although in use the surface would be non-planar) to illustrate how the femoral component is able to rotate and roll over the articulation surface of the tibial component, between extreme positions, during flexion and extension of a prosthetic knee assembly, in use. The curved outer surface 4 includes parting lines 10, 12, 14 formed on the outer surface at junctions defined between elements of an injection moulding tool used to mould the component 4, as described hereinafter with reference to Figures 9 to 13. Thus, the femoral component includes a first posterior parting line 10 formed on the first condyle 6, a second posterior parting line 12 formed on the second condyle 8, and a third anterior parting line 14 extending transversely across an anterior part of surface 4. In the figures, the height and width of the parting lines are exaggerated in the interests of clarity.

The parting lines 10, 12, 14 are positioned so the femoral component 2 can move between its extreme positions during flexion and extension (i.e. move through approximately 160°) without parting lines 10, 12, 14 (or for the avoidance of doubt any parting line associated with any part of the femoral component) contacting the articulation surface of the tibial component. Consequently, the only regions of the femoral component which contact the tibial component are "as-moulded" surfaces of the femoral component. Such surfaces can be moulded to have a low Ra. As-moulded articulation surfaces are preferred compared to surfaces which may be polished or otherwise treated to adjust their Ra (or remove parting lines or other undesirable features) since there is a risk, with any post-treatment, of articulation surfaces being contaminated, for example with metal from a tool used to effect a treatment or otherwise damaged during the process. In addition, avoiding post-treatment as described simplifies the manufacturing process for the femoral component which may make it quicker, easier and cheaper. Further details are provided below.

An internal face 16 of femoral component 2 includes respective undercut regions 18, 20 (Figures 2 and 9) associated with posterior and anterior edges of the femoral components. The undercut regions are adapted to facilitate retention of cement to aid securement of the femoral component into a femur of a patient being provided with a prosthetic knee. In addition, the internal face 16 includes a series of transverse ribs 19 which are also adapted to facilitate retention of cement as aforesaid.

Spaced apart projecting conical stems 22 extend inwardly away from the internal face 16, the stems being arranged to engage corresponding sockets formed in a patient's femur. In some embodiments, such stems may be omitted or may be a shape other than conical.

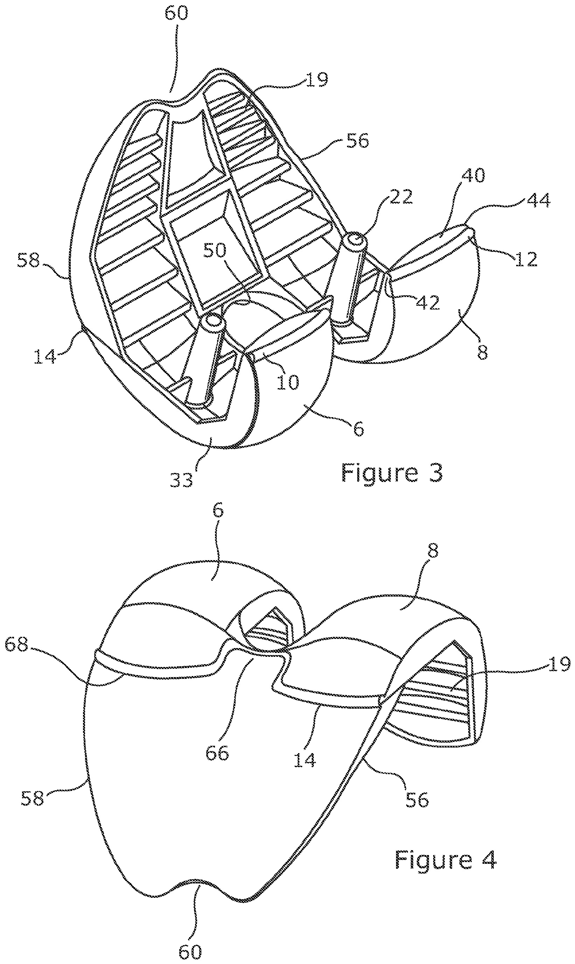

Referring to Figures 1 and 3, the first posterior parting line 10 has a height (above the adjacent outer surface 4) of 0.025mm and has a substantially constant cross-section along its extent. The parting line 10 extends the entire distance from one transverse side wall 33 to an opposite side wall 35 of the first condyle 6. The parting line 10 curves between its opposite ends to define an arc shape on the outer surface 4 which includes a concave edge 30 which faces a posterior end 32 of the first condyle 6. The concave edge 30 is situated at or close to posterior end 32. The maximum distance between the posterior end 32 and the concave edge 30 (distance 29 in Figure 1) may be less than 5mm. At the inner edge of concave edge 30 which faces the second condyle 8, the concave edge may be contiguous with the posterior end. It is suitably substantially a mirror image of concave edge 40 of the second condyle 8 which is shown, at position 42, to be contiguous with posterior end 44.

The second posterior parting line 12 on the second condyle is substantially a mirror image of the first posterior parting line 10 and the above description of the first posterior parting line applies to the second posterior parting line mutatis mutandis.

An opening 50 between the first and second condyles 6, 8 defines an intercondylar notch arranged to receive a stem 52 (Figure 6) provided on the tibial component 9. The intercondylar notch and stem are suitably arranged to restrict transverse movement of the femoral component 2 relative to the tibial component 9.

As shown in Figure 4, the third anterior parting line 14 extends the entire distance from transverse side 56 to transverse side 58, across a region of the outer surface 4 spaced from the first and second condyles in the direction of anterior end 60 of outer surface 4. The parting line 14 has a height (above outer surface 4) of 0.025mm and a substantially constant cross-section along its extent.

Figure 7 illustrates a patella 62. The patella 62 is in sliding engagement with the femur 64 via a projection (not shown) which extends between the first and second condyles 6, 8 within the intercondylar notch defined in opening 50. It is preferred that the parting line 14 does not contact the underside (or any part) of the patella during articulation of the prosthetic knee. To this end, a central curved region 66 (Figures 4 and 8) of parting line 14 retreats towards the intercondylar notch. Thus, as illustrated in Figure 8, parting line 14 includes a first region 68 (leftwards of dashed line 69) which has a substantially convex surface 75 facing towards the anterior end 60 of surface 4 and a substantially concave surface 77 facing towards first condyle 6; and a second region 79 (rightwards of dashed line 71) which has a substantially convex surface 8 facing towards the anterior end 60 and a substantially concave surface 81 facing towards second condyle 8. The central curved region 66 extending between dashed lines 69 and 71 incudes a substantially concave surface 82 facing towards the anterior end 60 of surface 4 and a substantially convex surface 83 adjacent the intercondylar notch. Thus, with the arrangement described, first and second regions 68, 79 are curved so as to optimise the length of the surface of the condyles 6, 8 which can contact the articulation surface of the tibial component without any of parting line 14 contacting the articulation surface. Furthermore the space available to receive the patella 62 such that its underside is not contacted by parting line 14 during articulation of the femoral component is optimised. It is preferred for curved region 66 to be as close to the perimeter of the intercondylar notch, defined by opening 50, as possible so to minimise the risk of it contacting the tibial component in use, as shown in Figure 4. In some embodiments, the shape 14 may be different than described, depending upon the shape of the femoral and/or tibial components.

As described, the femoral component is able to roll and/or rotate through a significant angle (e.g. 160° or more) over a surface of a tibial component without any parting line (e.g. parting lines 10, 12, 14) contacting the tibial component. Thus, potential wear on the tibial component by such parting lines is avoided.

Assessment (A) below provides a general method with reference to figure 5, for assessing the extent to which a femoral component can move over a planar surface without any parting line contacting the planar surface.

### Assessment (A) - Assessing extent of movement of femoral component over a planar surface without a parting line on the femoral component contacting the planar surface

Referring to figure 5, a femoral component 2 to be assessed is placed on a planar surface 86 of a table top 88 (or the like) with the component 2 initially in the position illustrated by arrow A. In this case, the component is arranged with first and second condyles 6, 8 contacting the surface and in a rotational position so parting line 12 just avoids contact with the surface. Similarly, although not shown in Figure 5, parting line 10 also avoids contact. Thus, position A represents one extreme position of the femoral component, wherein parting lines are very close to but do not contact the surface 86.

Next, the femoral component is pivoted and/or rolled linearly across the surface to the position illustrated by arrow B. It may suitably be moved from position A to position B about axis of rotation 90 of the femoral component which may be determined in accordance with ISO14243-1:2009(E). In position (B), component 2 is arranged so parting line 14 just avoids contact with the surface 86. Thus, position (B) represents another extreme position of the femoral component 2, wherein parting lines are very close to but do not contact the surface 86.

The angle through which femoral component moves between positions (A) and (B) can be assessed. In Figure 5, the angle is approximately 160°. This is referred to herein as the "normalized femoral rotation angle" (since it involves rotation across a planar surface). The normalized femoral rotation angle is also comparable to the angle through which the femoral component can move when engaged with a tibial component as illustrated in Figures 6 and 7. Thus, the femoral component 2 is arranged such that the positions A and B approximately correspond to the extreme positions of the femoral component when it moves over a corresponding tibial component between its positions of maximum flexion and extension. Movement of a femoral component over an actual tibial component may be assessed as described in Assessment B.

### Assessment (B) - Assessing extent of movement of femoral component over a tibial component in an assembly comprising the femoral component and tibial component

A femoral component and tibial component of a knee assembly for implantation are selected. The extent of movement can be assessed by assembling the components in vitro as shown in Figure 6 with the femoral component held at a first position (illustrated generally in Figure 6) which represents the extreme of flexion of the femoral compound on the tibial component, provided no parting line associated with the femoral component contacts the tibial component (although a parting line may be positioned very close to the tibial component). The femoral component may be rotated and/or rolled across the tibial component (as it would be when implanted) to a second position which represents the extreme of extension of the femoral component on the tibial component, provided no parting line associated with the femoral component contacts the tibial component at the first position, second position or any position between the first and second positions (although a parting line may be positioned very close to the tibial component, for example when in the second position). The angle through which the femoral component moves between the first and second positions (without any parting line on the femoral component contacting the tibial component) is referred to herein as the "practical femoral rotation angle" (since it is based on the angle achieved in an assembly which is suitable for and intended for implantation in a human body). The practical femoral rotation angle is typically in the range 140°-170° and may be patient dependent.

Thus, it should be appreciated that, advantageously, the femoral component can move through approximately up to 170° without any parting line on the femoral component contacting and potentially increasing the wear upon the tibial component. Thus, an assembly as described may have improved wear compared to assemblies which include parting lines at other positions on the femoral component.

It will be appreciated from the embodiments described that at angles greater than the normalized femoral rotation angle or the practical femoral rotation angle (e.g. when the femoral component is rotated beyond the first and second positions described) a parting line on the femoral component would contact the surface 86 (in Assessment A) or the surface of the tibial component (in Assessment B). However, this is not detrimental since the femoral component is not intended to be rotated beyond the first and second positions described. Nonetheless, by retaining a parting line produced in the manufacture of the femoral component as described, the femoral component may be more efficiently manufactured (since no additional parting line removal step is required) and the femoral component can be used substantially "as moulded", without the bearing surface of the femoral component being polished or otherwise treated to adjust its surface roughness, thereby obviating the risk of contaminating or damaging the articulation surface of the tibial component.

The femoral component 2 is injection moulded using virgin polyetheretherketone (PEEK) which may be PEEK-OPTIMA (Trade Mark), a long-term grade polyetheretherketone with a melt-viscosity of approximately 0.45 KNsm⁻², obtainable from Invibio Limited, UK.

The tibial component 9 is made from ultra-high molecular weight polyethylene (UHMWPE) which is softer than the PEEK. Consequently, steps are taken as described herein to minimise wear on the UHMWPE tibial component by the harder PEEK femoral component.

The position of parting lines (and areas which have no parting line) on the femoral component 2 has been described above. Such a femoral component 2 may be manufactured by injection moulding as hereinafter described with reference to figures 9 to 13.

Referring to Figure 9, a mould 90 for injection moulding the femoral component 2 comprises: a shell having a first element 92, a second element 94, and a third element 96; and a first inner up and way element 98, and a second inner up and away element 100.

The shell of the mould is arranged to define the outer surface 4 of the femoral component and the parting regions 10, 12, 14.

The second element 94 of the mould is arranged to define the entirety of the outer surface 4 of the femoral component which is arranged between parting lines 10, 12, 14. To this end, element 94 includes shaped surface 102 and first and second end faces 104, 106 which extend substantially parallel to one another. The shaped surface of the mould curves through an angle of about 180° to define the outer surface of the femoral component arranged between parting lines 10, 12, 14.

First element 92 cooperates with the second element 94 to define a first split line 108. To this end, first element 92 includes a shaped surface 110 and first and second end faces 112, 114. The first end face 112 abuts the end face 104 and defines part of the split line 108. The shaped surface 110 of element 92 curves through an angle of about 80° between its first and second end faces 112, 114,

The element 92 cooperates with first up and away element 98 to define a second split line 116 adjacent the proximal anterior flange of the femoral component.

Third element 96 comprises a central portion 97 located between said elements 98 and 100, and an arm portion 99. The arm portion 99 is joined to the central portion 97 such that movement of element 96 causes simultaneous movement of said portions 97 and 99. The arm portion 99 cooperates and makes face to face contact with second end face 106 of outer element 94 to define respective split lines 118, 120 adjacent superior posterior condyles of the femoral component 4.

The first, second and third up and away elements 96, 98, 100 cooperate to define the internal face 16 of the femoral component including undercut regions 18, 20.

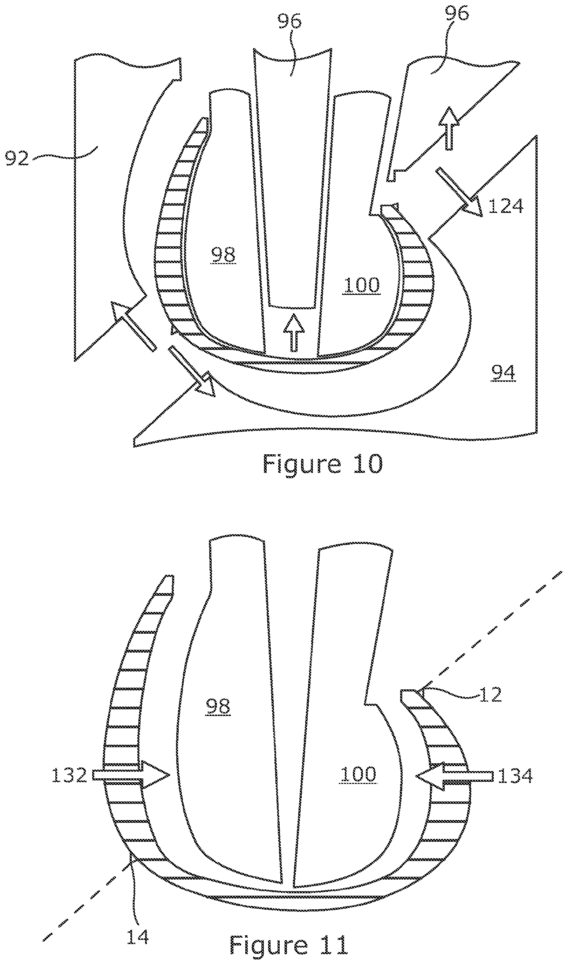

The mould of Figure 9 is injected with molten PEEK to fill the mould. After allowing the PEEK to cool, the moulded femoral component can be removed from the mould. Referring to Figure 9, initially, element 96 moves up as indicated by arrow 101. The outer shell of the mould is then removed by moving elements 92 in the direction of arrow 122, and the second element 94 in the direction of arrows 124. This is shown most clearly in Figure 10.

The removal of elements 92, 94, 96 causes the second and third up and away elements 98, 100 to move inwardly towards one another as represented by arrow 132, 134 in Figure 11 and disengage from the femoral component 2.

Consequently, the parting lines are thus formed at locations on the surface of the component which do not obstruct use, or cause damage to a corresponding mating surface. Most advantageously, the moulding tool assembly results in a component having parting lines in favourable locations.

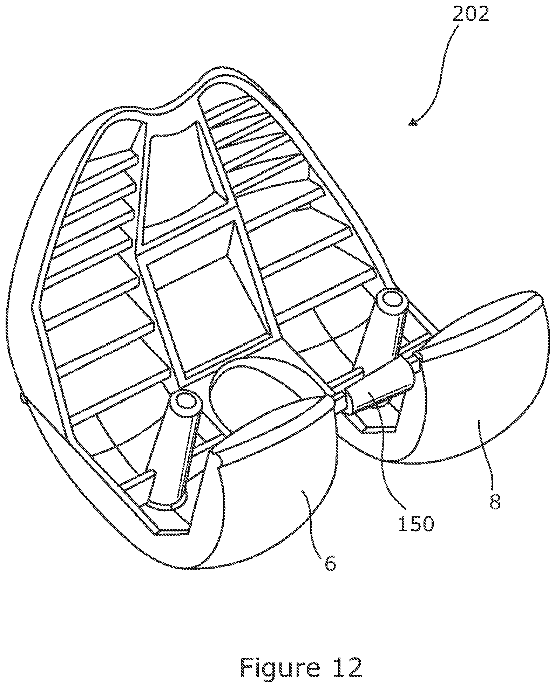

As an alternative to the Figure 3 arrangement which is a design wherein a patient's cruciate ligament is retained and an opening 50 defines an intercondylar notch in which the cruciate ligament is positioned, the femoral component may include a bridge 150 which extends between first and second condyles 6, 8, as shown in Figure 12 which illustrates a posterior stabilised arrangement.

The invention is not restricted to the details of the foregoing embodiments and is defined by the appended claims.

## Claims

1. A femoral component (2) of a knee prosthesis, wherein said component is a one piece injection moulded component, said component comprising a curved outer surface (4) for bearing against a tibial component, said curved outer surface (4) including a posterior end (32) and an anterior end (60), said curved outer surface (4) including an area (A) which extends from a first position closer to the posterior end (32) to a second position closer to the anterior end (60), wherein said area (A) includes no parting line (10, 12, 14) formed on the outer surface at junctions defined between elements of an injection moulding tool used to mould the component, wherein area (A) suitably comprises an area of the curved outer surface which is arranged to contact, pivot and/or roll over a tibial surface of a tibial component in normal use, **characterised in that** said component is arranged to pivot through an angle of at least 140° on a planar surface (86) without any parting line and/or without any remnant of a parting line contacting the planar surface (86).

2. A femoral component as claimed in claim 1, wherein area (A) suitably is an area of said curved outer surface (4) of said femoral component (2) which has not been treated to remove or reduce any parting line.

3. A femoral component as claimed in any one of the preceding claims, wherein the shape of area (A) is preferably wholly defined by a tool surface used in its manufacture, for example by a surface of an injection moulding tool.

4. A femoral component as claimed in any one of the preceding claims, wherein Area (A) is a wholly as-moulded surface.

5. A femoral component as claimed in claim any one of the preceding claims, wherein said femoral component (2) includes said curved outer surface (4) for bearing against the tibial component and a flexion/extension axis as described in ISO14243-1:2009(E) at 3.6, wherein Area (A) of said curved outer surface suitably subtends an angle of at least 150° with the flexion/extension axis.

6. A femoral component as claimed in claim any one of the preceding claims, wherein said femoral component (2) is arranged to pivot through an angle of at least 150° or at least 155°, without any parting line and/or without any remnant of a parting line contacting the planar surface (86).

7. A femoral component as claimed in any one of the preceding claims, wherein said femoral component (2) includes an undercut region (18, 20), wherein said undercut region (18, 20) is defined in an internal face (16) of said femoral component (2) which faces in a direction which is opposite to the direction in which said outer surface (4) faces.

8. A femoral component as claimed in claim 7, wherein a series of ribs (19) are provided in said internal face (16) of the femoral component (2), wherein the ribs (19) are equi-distantly spaced, preferably running parallel to one another.

9. A femoral component as claimed in claim 8, wherein the ribs (19) run parallel to the flexion extension axis.

10. A femoral component as claimed in any one of the preceding claims, wherein said femoral component (2) comprises a polymeric material,wherein the polymeric material is a polyaryletherketone, preferably polyetheretherketone.

11. A combination for a knee prosthesis, the combination comprising a femoral componen (2) according to any one of the preceding claims and a tibial component (9), wherein the femoral component (2) comprises polyaryletherketone and the tibial component (9) comprises a second polymer, preferably a polyolefin, for example polyethylene, a polyurethane or a polyamide.

12. A combination as claimed in claim 11, wherein the second polymer comprises UHMWPE.

13. A tooling apparatus configured for moulding a femoral component (2) as claimed in any one of the preceding claims, the tooling apparatus comprising a mould for injection moulding the femoral component (2), the mould having a first element, a second element, a third element, and at least one up and away element, wherein the mould is operable such that parting lines are formed at locations on the surface of the component which do not obstruct use, or cause damage to a corresponding mating surface.

14. A method of making a femoral component (2) according any one of the preceding claims which comprises injection moulding a thermoplastic polymeric material, for example comprising said first polymer to form said femoral component (2).

## Patentansprüche

1. Femurkomponente (2) einer Knieprothese, wobei die Komponente eine einteilige spritzgegossene Komponente ist, die Komponente umfassend eine gekrümmte Außenoberfläche (4) zum Liegen gegen eine Tibiakomponente, die gekrümmte Außenoberfläche (4) einschließlich eines hinteren Endes (32) und eines vorderen Endes (60), die gekrümmte Außenoberfläche (4) einschließlich eines Bereichs (A), der sich von einer ersten Position näher an dem hinteren Ende (32) zu einer zweiten Position näher an dem vorderen Ende (60) erstreckt, wobei der Bereich (A) keine Trennlinie (10, 12, 14) einschließt, die an der Außenoberfläche an Verbindungsstellen ausgebildet ist, die zwischen Elementen eines Spritzgießwerkzeugs definiert sind, die verwendet werden, um die Komponente zu formen, wobei der Bereich (A) geeigneterweise einen Bereich der gekrümmten Außenoberfläche umfasst, der angeordnet ist, um eine Tibiaoberfläche einer Tibiakomponente in normaler Verwendung zu berühren, über sie zu schwenken und/oder zu rollen, **dadurch gekennzeichnet, dass**, die Komponente angeordnet ist, um durch einen Winkel von mindestens 140° auf einer planaren Oberfläche (86) zu schwenken, ohne dass eine beliebige Trennlinie und/oder ohne dass ein beliebiger Rest einer Trennlinie die planare Oberfläche (86) berührt.

2. Femurkomponente nach Anspruch 1, wobei der Bereich (A) geeigneterweise ein Bereich der gekrümmten Außenoberfläche (4) der Femurkomponente (2) ist, der nicht behandelt wurde, um eine beliebige Trennlinie zu entfernen oder zu reduzieren.

3. Femurkomponente nach einem der vorstehenden Ansprüche, wobei die Gestalt des Bereichs (A) vorzugsweise durch eine Werkzeugoberfläche vollständig definiert ist, die in ihrer Herstellung verwendet wird, zum Beispiel durch eine Oberfläche eines Spritzgießwerkzeugs.

4. Femurkomponente nach einem der vorstehenden Ansprüche, wobei der Bereich (A) eine vollständig wie geformte Oberfläche ist.

5. Femurkomponente nach einem der vorstehenden Ansprüche, wobei die Femurkomponente (2) die gekrümmte Außenoberfläche (4) zum Liegen gegen die Tibiakomponente und eine Beugungs-/Streckungsachse einschließt, wie in ISO14243-1:2009(E) bei 3.6 beschrieben, wobei der Bereich (A) der gekrümmten Außenoberfläche geeigneterweise einen Winkel von mindestens 150° mit der Beugungs-/Streckungsachse begrenzt.

6. Femurkomponente nach einem der vorstehenden Ansprüche, wobei die Femurkomponente (2) angeordnet ist, um durch einen Winkel von mindestens 150° oder mindestens 155°, ohne dass eine beliebige Trennlinie, und/oder ohne dass ein beliebiger Rest einer Trennlinie die planare Oberfläche (86) berührt, zu schwenken.

7. Femurkomponente nach einem der vorstehenden Ansprüche, wobei die Femurkomponente (2) eine Unterschneidungsregion (18, 20) einschließt, wobei die Unterschneidungsregion (18, 20) in einer Innenfläche (16) der Femurkomponente (2) definiert ist, die in eine Richtung weist, die der Richtung gegenüberliegt, in die die Außenoberfläche (4) weist.

8. Femurkomponente nach Anspruch 7, wobei eine Reihe von Rippen (19) in der Innenfläche (16) der Femurkomponente (2) bereitgestellt sind, wobei die Rippen (19) äquidistant beabstandet sind, vorzugsweise parallel zueinander verlaufen.

9. Femurkomponente nach Anspruch 8, wobei die Rippen (19) parallel zu der Beugungsstreckungsachse verlaufen.

10. Femurkomponente nach einem der vorstehenden Ansprüche, wobei die Femurkomponente (2) ein Polymermaterial umfasst, wobei das Polymermaterial ein Polyaryletherketon, vorzugsweise Polyetheretherketon, ist.

11. Kombination für eine Knieprothese, die Kombination umfassend eine Femurkomponente (2) nach einem der vorstehenden Ansprüche und eine Tibiakomponente (9), wobei die Femurkomponente (2) Polyaryletherketon umfasst und die Tibiakomponente (9) ein zweites Polymer, vorzugsweise ein Polyolefin, zum Beispiel Polyethylen, ein Polyurethan oder ein Polyamid umfasst.

12. Kombination nach Anspruch 11, wobei das zweite Polymer UHMWPE umfasst.

13. Werkzeugeinrichtung zum Formen einer Femurkomponente (2) nach einem der vorstehenden Ansprüche, die Werkzeugeinrichtung umfassend eine Form zum Spritzgießen der Femurkomponente (2),
wobei die Form ein erstes Element, ein zweites Element, ein drittes Element und mindestens ein Hoch- und Weg-Element aufweist, wobei die Form derart betreibbar ist, dass Trennlinien an Stellen auf der Oberfläche der Komponente ausgebildet sind, die die Verwendung nicht behindern oder keine Beschädigung einer entsprechenden Passoberfläche verursachen.

14. Verfahren zum Anfertigen einer Femurkomponente (2) nach einem der vorstehenden Ansprüche, das das Spritzgießen eines thermoplastischen Polymermaterials umfasst, zum Beispiel umfassend das erste Polymer, um die Femurkomponente (2) auszubilden.

## Revendications

1. Composant fémoral (2) d'une prothèse de genou, dans lequel ledit composant est un composant moulé par injection d'un seul tenant, ledit composant comprenant une surface extérieure incurvée (4) pour un appui contre un composant tibial, ladite surface extérieure incurvée (4) comportant une extrémité postérieure (32) et une extrémité antérieure (60), ladite surface extérieure incurvée (4) comportant une zone (A) qui s'étend d'une première position plus proche de l'extrémité postérieure (32) vers une seconde position plus proche de l'extrémité antérieure (60), ladite zone (A) ne comportant aucune ligne de séparation (10, 12, 14) formée sur la surface extérieure au niveau de jonctions définies entre des éléments d'un outil de moulage par injection utilisé pour mouler le composant, la zone (A) comprenant de manière appropriée une zone de la surface extérieure incurvée qui est agencée pour entrer en contact, pivoter et/ou rouler sur une surface tibiale d'un composant tibial lors d'une utilisation normale, **caractérisé en ce que** le dit composant est agencé pour pivoter à un angle d'au moins 140 ° sur une surface plane (86) sans aucune ligne de séparation et/ou sans aucun reste d'une ligne de séparation entrant en contact avec la surface plane (86).

2. Composant fémoral selon la revendication 1, dans lequel la zone (A) est de manière appropriée une zone de ladite surface extérieure incurvée (4) dudit composant fémoral (2) qui n'a pas été traitée pour retirer ou réduire toute ligne de séparation.

3. Composant fémoral selon l'une quelconque des revendications précédentes, dans lequel la forme de la zone (A) est de préférence entièrement définie par une surface d'outil utilisée dans sa fabrication, par exemple par une surface d'un outil de moulage par injection.

4. Composant fémoral selon l'une quelconque des revendications précédentes, dans lequel la zone (A) est une surface entièrement moulée.

5. Composant fémoral selon l'une quelconque des revendications précédentes, dans lequel ledit composant fémoral (2) comporte ladite surface extérieure incurvée (4) pour un appui contre le composant tibial et un axe de flexion/extension tel que décrit dans ISO14243-1:2009(E) à 3,6, la zone (A) de ladite surface extérieure incurvée sous-tendant de manière appropriée un angle d'au moins 150 ° avec l'axe de flexion/extension.

6. Composant fémoral selon l'une quelconque des revendications précédentes, dans lequel ledit composant fémoral (2) est agencé pour pivoter à un angle d'au moins 150 ° ou d'au moins 155 °, sans aucune ligne de séparation et/ou sans aucun reste d'une ligne de séparation entrant en contact avec la surface plane (86).

7. Composant fémoral selon l'une quelconque des revendications précédentes, dans lequel ledit composant fémoral (2) comporte une région en contre-dépouille (18, 20), ladite région en contre-dépouille (18, 20) étant définie dans une face interne (16) dudit composant fémoral (2) qui fait face dans une direction qui est opposée à la direction dans laquelle lesdites faces de surface extérieure (4) sont opposées.

8. Composant fémoral selon la revendication 7, dans lequel une série de nervures (19) sont fournies dans ladite face interne (16) du composant fémoral (2), les nervures (19) étant espacées de manière équidistante, de préférence s'étendant parallèlement les unes aux autres.

9. Composant fémoral selon la revendication 8, dans lequel les nervures (19) s'étendent parallèlement à l'axe d'extension de flexion.

10. Élément fémoral selon l'une quelconque des revendications précédentes, dans lequel ledit composant fémoral (2) comprend un matériau polymère, le matériau polymère étant une polyaryléthercétone, de préférence du polyétheréthercétone.

11. Combinaison pour une prothèse de genou, la combinaison comprenant un composant fémoral (2) selon l'une quelconque des revendications précédentes et un composant tibial (9), le composant fémoral (2) comprenant de la polyaryléthercétone et le composant tibial (9) comprenant un second polymère, de préférence une polyoléfine, par exemple du polyéthylène, un polyuréthane ou un polyamide.

12. Combinaison selon la revendication 11, dans laquelle le second polymère comprend du UHMWPE.

13. Appareil d'outillage configuré pour mouler un composant fémoral (2) selon l'une quelconque des revendications précédentes, l'appareil d'outillage comprenant un moule pour mouler par injection le composant fémoral (2),
le moule ayant un premier élément, un deuxième élément, un troisième élément et au moins un élément en hauteur et éloigné, le moule pouvant fonctionner de telle sorte que des lignes de séparation sont formées à des emplacements sur la surface du composant qui n'obstrue pas d'utilisation ou entraînent des dommages à une surface d'accouplement correspondante.

14. Procédé de fabrication d'un composant fémoral (2) selon l'une quelconque des revendications précédentes, qui comprend le moulage par injection d'un matériau polymère thermoplastique, comprenant par exemple ledit premier polymère pour former ledit composant fémoral (2).
